# EUROPÄISCHE PATENTSCHRIFT

(11) **EP 0 609 179 B1**
(45) Veröffentlichungstag und Bekanntmachung des Hinweises auf die Patenterteilung: **17.07.1996**
(21) Anmeldenummer: 94810034.2
(22) Anmeldetag: 26.01.1994
(51) Int. Cl.: C07C 253/00, C07C 255/49

(54) **Herstellung aromatischer Nitrile**
Preparation of aromatic nitriles
Procédé de préparation de nitriles aromatiques

(30) Priorität: 28.01.1993 CH 238/93
(43) Veröffentlichungstag der Anmeldung: 03.08.1994
(73) Patentinhaber: CIBA-GEIGY AG, 4002 Basel (CH)
(72) Erfinder: Güleç, Bilge, Dr., CH-4058 Basel (CH)

(56) Entgegenhaltungen:
- EP-A- 0 080 700
- GB-A- 2 198 728
- US-A- 4 235 807
- CHEMICAL ABSTRACTS, vol. 99, no. 7, 15. August 1983, Columbus, Ohio, US; abstract no. 53294z, C.I. CHIRIAC

## Beschreibung

Die vorliegende Anmeldung betrifft die Herstellung von aromatischen Nitrilen durch Umsetzung entsprechender Aldehyde mit Hydroxylaminsulfat in Propionsäure in Anwesenheit eines Propionsäuresalzes.

Die Umsetzung von Aldehyden mit Hydroxylaminsalzen und anschliessender Dehydratisierung des erhaltenen Oxims zum Nitril ist seit langem bekannt. Zur Dehydratisierung wurden verschiedene Methoden vorgeschlagen, so z.B. in C.A. 85, 93176e (1976) durch Erhitzen in Dimethylformamid, in EP-B 80700 durch azeotropes Abdestillieren des Wassers mit Hilfe eines ein azeotropes Gemisch bildenden und mit Wasser nicht mischbaren Lösungsmittels, in Synthesis 1979, 2, 112-113 und in Huaxue Shiji 1990, 12(5), 314, 292 durch Erhitzen in Ameisensäure, in Journal of Nanjing Univ. 1990, 26(2), 263-266, durch Erhitzen in Ameisensäure oder Eisessig und in J. Chem. Soc. 1933, IX, 43 durch Erhitzen in Essigsäureanhydrid. Von einer Verwendung von Dimethylformamid oder von mit Wasser nicht mischbaren Lösungsmitteln im grosstechnischen Massstab ist heutzutage aus toxischen und umweltpolitischen Ueberlegungen wenn möglich abzusehen. Das Arbeiten mit Ameisensäure führt wohl zu sehr guten Resultaten, ist aber wegen der starken ätzenden Wirkung, der Toxizität und der aufwendigen Regenerierung nicht ratsam. Mit dem Einsatz von Eisessig hätte man einige dieser Nachteile beheben können, doch hat sich herausgestellt, dass bestimmte Benzonitrile damit nur mit einer sehr schlechten Ausbeute erhalten werden können.

Es ist nun gefunden worden, dass durch den Einsatz einer schwächeren Säure, wie die Propionsäure, dieselben Nitrile ganz überraschend mit einer erheblich besseren und durchaus zufriedenstellenden Ausbeute erhalten werden können. Dies ist umso verwunderlicher in Anbetracht der Lehre aus dem US Patent 4 235 807, wonach die Umsetzung eines Benzaldehyds mit Hydroxylamin zum Benzonitril unter Verwendung einer organischen Säure (u.a. z.B. auch Propionsäure) als Lösungsmittel nur in Gegenwart eines Dehydrierungsmittels, wie Essigsäure- oder Propionsäureanhydrid, Phosphorpentoxid oder Dimethylacetal, stattfindet. Dadurch können die obenerwähnten Nachteile weitgehend vermieden werden, denn die Propionsäure ist toxisch unbedenklich, viel weniger ätzend als Ameisensäure und kann problemlos regeneriert werden.

Die vorliegende Anmeldung betrifft demnach ein Verfahren zur Herstellung eines Nitrils der Formel 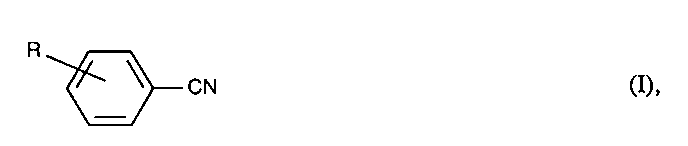
worin R, Wasserstoff, C₁-C₄-Alkyl oder Phenyl bedeutet, durch Umsetzung eines Aldehyds der Formel 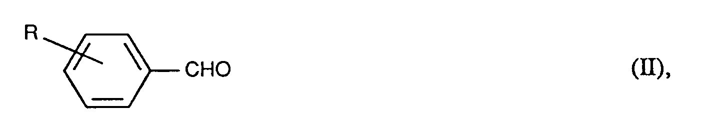
worin R die oben angegebene Bedeutung hat, mit Hydroxylaminsulfat und nachfolgende Dehydratisierung, dadurch gekennzeichnet, dass die Umsetzung in Propionsäure in Gegenwart eines Propionsäuresalzes durch Erhitzen auf eine Aussentemperatur von 140 bis 165°C, bevorzugt 150 bis 160°C, während 2 bis 6, bevorzugt 3 bis 5 Stunden, bei Atmosphärendruck unter gleichzeitigem Abdestillieren des sich bildenden Propionsäure/Wasser-Gemisches, bis zur Bildung einer Lösung und nachfolgendem abschliessendem Abdestillieren des Propionsäure/Wasser-Gemisches bei 100-130°C, bevorzugt 110 bis 120°C, im Vakuum durchgeführt wird und danach das erhaltene Nitril nach allgemein üblichen Methoden isoliert wird.

Die Umsetzung erfolgt nach folgendem Reaktionsschema: 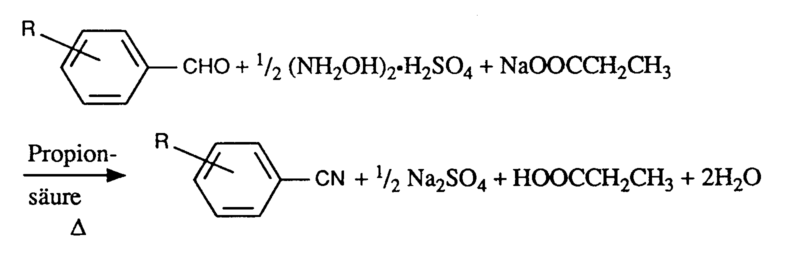
R bedeutet als C₁-C₄-Alkyl Methyl, Ethyl, n-Propyl, Isopropyl, n-Butyl, sec.-Butyl oder tert.-Butyl.

Als Propionsäuresalz kann zweckmässig ein Alkalimetallsalz, insbesondere Na-Propionat eingesetzt werden.

R ist bevorzugt in der 4-Stellung und bedeutet insbesondere tert.-Butyl oder ganz bevorzugt Phenyl.

Bei den Aldehyden der Formel II handelt es sich um bekannte Verbindungen.

Die Propionsäure wird zweckmässig in einer Menge von 10 bis 20, bevorzugt 15 bis 18 Mol, bezogen auf 1 Mol Aldehyd eingesetzt.

Hydroxylaminsulfat wird annähernd in stöchiometrischer Menge, bevorzugt mit leichtem Ueberschuss, d.h. 0,505 bis 0,58 Mol auf 1 Mol Aldehyd, eingesetzt.

Für das Na-Propionat ist eine Menge von 1,05 bis 1,3 Mol, bezogen auf 1 Mol Hydroxylaminsulfat, zweckmässig.

Die durch das erfindungsgemässe Verfahren erhältlichen Nitrile sind wertvolle Zwischenprodukte u.a. für die Herstellung von Diketopyrrolopyrrolpigmenten.

Die nachfolgenden Beispiele erläutern die Erfindung.

### Beispiel 1:

606,0 g (8,2 Mol) Propionsäure werden in einem Sulfierkolben bei Raumtemperatur vorgelegt und 91,10 g (0,5 Mol) 4-Biphenylaldehyd, 28,82 g (0,3 Mol) Natriumpropionat und 45,14 g (0,28 Mol) Hydroxylaminsulfat zugegeben. Dieses Gemisch wird innerhalb 30 Minuten auf 153°C Aussentemperatur erhitzt und danach 3 Stunden bei dieser Temperatur unter Normaldruck gerührt. Während dieser Zeit destilliert man ein Lösungsmittelgemisch, das aus Propionsäure und Wasser besteht, ständig ab. Nach dreistündiger Destillation entsteht eine Lösung.

Anschliessend wird die Aussentemperatur auf 120°C herabgesetzt, Vakuum angelegt und während 2 Stunden bei dieser Temperatur und einem Vakuum von ca. 390 mbar ein Lösungsmittelgemisch, bestehend aus Propionsäure und Wasser abdestilliert.

Danach wird das Vakuum entlastet, der Rückstand annähernd auf Raumtemperatur abgekühlt, durch ausgiessen auf Eis/Wasser ausgefällt, filtriert und mit Wasser neutral gewaschen. Die Rohausbeute beträgt 89,3 g, mit einem Gehalt an reinem 4-Biphenylnitril von 85,7 % (ca 85 % d.Th).

### Beispiel 2:

606,0 g (8,2 Mol) Propionsäure werden in einem Sulfierkolben bei Raumtemperatur vorgelegt, und 81,15 g (0,5 Mol) 4-tert.-Butylbenzaldehyd, 28,82 g (0,3 Mol) Natriumpropionat und 45,14 g (0,28 Mol) Hydroxylaminsulfat zugegeben. Dieses Gemisch wird innerhalb 30 Minuten auf 158°C Aussentemperatur erhitzt und danach 5 Stunden bei dieser Temperatur unter Normaldruck gerührt. Während dieser Zeit destilliert man ein Lösungsmittelgemisch, das aus Propionsäure und Wasser besteht, ständig ab. Nach fünfstündiger Destillation entsteht eine Lösung.

Anschliessend wird die Aussentemperatur auf 120°C herabgesetzt, Vakuum angelegt und während 2 Stunden bei dieser Temperatur und einem Vakuum von ca. 390 mbar ein Lösungsmittelgemisch, bestehend aus Propionsäure und Wasser abdestilliert.

Danach wird das Vakuum entlastet, die Aussentemperatur auf 100°C herabgesetzt, wiederum 28,82 g Natriumpropionat zugegeben und unter Normaldruck 15 Minuten ausgerührt.

Nach Beendigung des Ausrührens wird ein Vakuum von ca. 50-65 mbar angelegt, auf 120°C Aussentemperatur erwärmt und die restliche Propionsäure abdestilliert.

Der ölige Rückstand wird bei Raumtemperatur mit Natriumbicarbonat und Wasser neutral gewaschen anschliessend wird die organische Phase abgetrennt. Die Rohausbeute beträgt 83,9 g, mit einem Gehalt an reinem 4-tert.-Butylbenzonitril von 71,1 % (ca 74 % d.Th).

### Beispiel 3:

370,4 g (5 Mol) Propionsäure werden in einem 1 l Sulfierkolben bei Raumtemperatur vorgelegt, und 58,58 g (0,5 Mol) 4-Methyl-benzaldehyd, 14,55 g (0,15 Mol) Natriumpropionat und 45,14 g (0,28 Mol) Hydroxylaminsulfat zugegeben. Dieses Gemisch wird innerhalb 30 Minuten auf 153°C (Aussentemperatur) erhitzt und ca. 10 Minuten bei dieser Temperatur unter Normaldruck gerührt. Nach zehnminütigem Rühren entsteht eine Lösung.

Anschliessend wird die Aussentemperatur auf 120°C herabgesetzt, Vakuum angelegt und 4 Stunden bei dieser Temperatur und einem Vakuum von ca. 400 mbar ein Lösungsmittelgemisch, bestehend aus Propionsäure und Wasser, abdestilliert.

Danach wird das Vakuum entlastet, die Aussentemperatur auf 100°C herabgesetzt, 48,7 g (0,51 Mol) Natriumpropionat zugegeben und unter Normaldruck 15 Minuten ausgerührt.

Nach Beendigung des Ausrührens wird das Reaktionsgemisch auf Raumtemperatur abgekühlt, über eine Glasfritte abfiltriert. Die Ausbeute an 4-Methyl-benzonitril beträgt 46,07 g (78,65 % d.Th.).

### Beispiel 4:

606,0 g (8,2 Mol) Propionsäure werden in einem 1 l Sulfierkolben bei Raumtemperatur vorgelegt, und 53,06 g (0,5 Mol) Benzaldehyd, 14,55 g (0,15 Mol) Natriumpropionat und 45,14 g (0,28 Mol) Hydroxylaminsulfat zugegeben. Dieses Gemisch wird innerhalb 30 Minuten auf 153°C (Aussentemperatur) erhitzt und ca. 20 Minuten bei dieser Temperatur unter Normaldruck gerührt. Nach zwanzigminütigem Rühren entsteht eine Lösung.

Anschliessend wird die Aussentemperatur auf 120°C herabgesetzt, Vakuum angelegt und 4 Stunden bei dieser Temperatur und einem Vakuum von ca. 400 mbar ein Lösungsmittelgemisch, bestehend aus Propionsäure und Wasser, abdestilliert.

Danach wird das Vakuum entlastet, die Aussentemperatur auf 100°C herabgesetzt, 48,7 g (0,51 Mol) Natriumpropionat zugegeben und unter Normaldruck 15 Minuten ausgerührt.

Nach Beendigung des Ausrührens wird das Reaktionsgemisch auf Raumtemperatur abgekühlt, über eine Glasfritte abfiltriert. Die Ausbeute an Benzonitril beträgt 42,11 g (81,7 % d.Th.).

## Patentansprüche

1. Verfahren zur Herstellung eines Nitrils der Formel 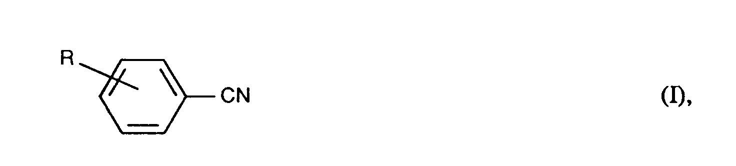 worin R, Wasserstoff, C₁-C₄-Alkyl oder Phenyl bedeutet, durch Umsetzung eines Aldehyds der Formel 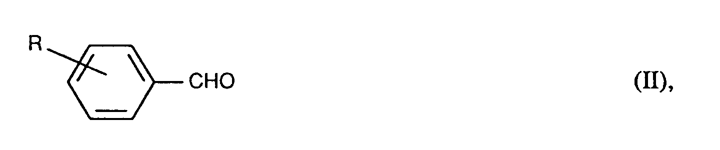 worin R die oben angegebene Bedeutung hat, mit Hydroxylaminsulfat und nachfolgende Dehydratisierung, dadurch gekennzeichnet, dass die Umsetzung in Propionsäure in Gegenwart eines Propionsäuresalzes durch Erhitzen auf eine Aussentemperatur von 140 bis 165°C, bevorzugt 150 bis 160°C, während 2 bis 6, bevorzugt 3 bis 5 Stunden, bei Atmosphärendruck unter gleichzeitigem Abdestillieren des sich bildenden Propionsäure/Wasser-Gemisches, bis zur Bildung einer Lösung und nachfolgendem abschliessendem Abdestillieren des Propionsäure/Wasser-Gemisches bei 100-130°C, bevorzugt 110 bis 120°C, im Vakuum durchgeführt wird und danach das erhaltene Nitril nach allgemein üblichen Methoden isoliert wird.

2. Verfahren gemäss Anspruch 1, dadurch gekennzeichnet, dass R in 4-Stellung steht.

3. Verfahren gemäss Anspruch 2, dadurch gekennzeichnet, dass R tert.-Butyl oder Phenyl bedeutet.

4. Verfahren gemäss Anspruch 2, dadurch gekennzeichnet, dass R Phenyl bedeutet.

5. Verfahren gemäss Anspruch 1, dadurch gekennzeichnet, dass die Propionsäure in einer Menge von 10 bis 20 Mol, bezogen auf ein Mol Aldehyd eingesetzt wird.

6. Verfahren gemäss Anspruch 5, dadurch gekennzeichnet, dass das Hydroxylaminsulfat in einer Menge von 0,505 bis 0,55 Mol auf 1 Mol Aldehyd und das Na-Propionat in einer Menge von 1,05 bis 1,3 Mol auf 1 Mol Hydroxylaminsulfat eingesetzt werden.

## Claims

1. A process for the preparation of a nitrile of formula 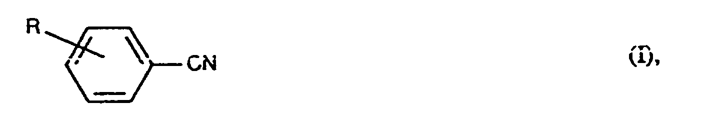 wherein R is hydrogen, C₁-C₄alkyl or phenyl, by reaction of an aldehyde of formula 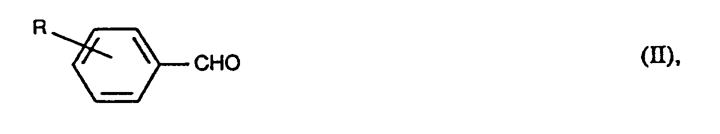 wherein R is as defined above, with hydroxylamine sulfate and subsequent dehydration, which process comprises carrying out the reaction in propionic acid in the presence of a salt of propionic acid by heating to an external temperature of from 140 to 165#C, preferably from 150 to 160#C, for from 2 to 6 hours, preferably from 3 to 5 hours, under atmospheric pressure and simultaneously distilling off the mixture of propionic acid and water which is produced until a solution forms, and thereafter removing the mixture of propionic acid and water at 100-130#C, preferably from 110 to 120#C by vacuum distillation, and then isolating the resultant nitrile by standard methods.

2. A process according to claim 1, wherein R is in position 4.

3. A process according to claim 2, wherein R is tert-butyl or phenyl.

4. A process according to claim 2, wherein R is phenyl.

5. A process according to claim 1, wherein the propionic acid is employed in an amount of 10 to 20 mol, based on one mole of aldehyde.

6. A process according to claim 5, wherein the hydroxylamine sulfate is employed in an amount of 0.505 to 0.55 mol per mole of aldehyde, and Na propionate is employed in an amount of 1.05 to 1.3 mol per mole of hydroxylamine sulfate.

## Revendications

1. Procédé pour préparer un nitrile de formule : 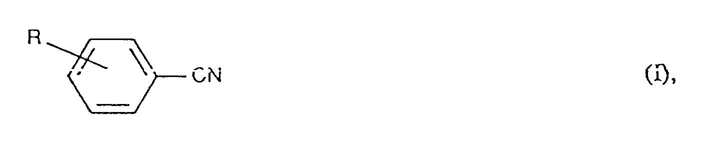 dans laquelle R est un hydrogène ou un groupe alkyle en C₁-C₄ ou phényle, par réaction d'un aldéhyde de formule : 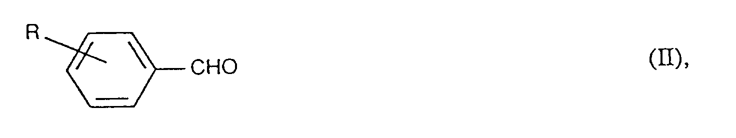 dans laquelle R a les significations données ci-dessus, avec du sulfate d'hydroxylamine, suivie d'une déshydratation, caractérisé en ce que la réaction est mise en oeuvre sous vide dans de l'acide propionique, en présence d'un sel de l'acide propionique, par chauffage à une température extérieure de 140 à 165°C, de préférence de 150 à 160°C, pendant 2 à 6 et de préférence pendant 3 à 5 heures, sous la pression atmosphérique, tout en chassant simultanément par distillation le mélange acide propionique/eau qui se forme, jusqu'à formation d'une solution, puis élimination, par distillation, du mélange acide propionique/eau à une température de 100 à 130°C et de préférence de 110 à 120°C, le nitrile obtenu étant ensuite isolé par des procédés usuels.

2. Procédé selon la revendication 1, caractérisé en ce que R est en position 4.

3. Procédé selon la revendication 2, caractérisé en ce que R est le groupe tert-butyle ou phényle.

4. Procédé selon la revendication 2, caractérisé en ce que R est le radical phényle.

5. Procédé selon la revendication 1, caractérisé en ce que l'acide propionique est utilisé en une quantité de 10 à 20 moles par mole d'aldéhyde.

6. Procédé selon la revendication 5, caractérisé en ce que le sulfate d'hydroxylamine est utilisé en une quantité de 0,505 à 0,55 mole par mole d'aldéhyde et le propionate de Na est utilisé en une quantité de 1,05 à 1,3 mole par mole de sulfate d'hydroxylamine.
